# EUROPEAN PATENT APPLICATION

(11) **EP 4 173 562 A1**
(43) Date of publication of application: **03.05.2023**
(21) Application number: 21828860.3
(22) Date of filing: 21.06.2021
(51) Int. Cl.: A61B 5/0537

(54) **MEASUREMENT DEVICE**

(30) Priority: 25.06.2020 JP 2020109877
(71) Applicant: Tanita Corporation, Tokyo 174-8630 (JP)
(72) Inventor: KODAMA, Miyuki, Tokyo 174-8630 (JP); NAGAHAMA, Toshiki, Tokyo 174-8630 (JP); SAKAI, Yoshio, Tokyo 174-8630 (JP); TANIDA, Senri, Tokyo 174-8630 (JP)
(74) Representative: Eisenführ Speiser
(86) International application number: PCT/JP2021/023438
(87) International publication number: WO 2021/261454

(57) **Abstract**

A purpose of the disclosure is to provide a measurement device for human bodies that can encourage a person who is reluctant to measure body composition to use the device. A measurement device has: an electric resistance value acquisition unit for acquiring an electric resistance value of one or more parts of a body of a person to be measured; and a type determination unit for using the electric resistance value to determine a type representing a physical characteristic of the person to be measured from one or more predetermined points of view.

## Description

### CROSS-REFERENCE TO RELATED APPLICATION

This application claims the benefit of Japanese Patent Application No. 2020-109877 filed on June 25, 2020 in Japan, the contents of which are incorporated herein by reference.

### TECHNICAL FIELD

This disclosure relates to a measurement device for human bodies.

### BACKGROUND ART

Measurement devices using BIA (Bioelectrical Impedance Analysis, i.e., bioimpedance analysis) are known as a way to measure human body composition (e.g., Patent document 1). Such measurement devices estimate (measure) body composition by applying a small current to a body and measuring its electric resistance value. This estimation requires the height, weight, or the like of a person to be measured, and is desirably provided with information on the person's gender, age, or the like.

### PRIOR ART DOCUMENT

### Patent document

Patent document 1: Japanese Patent Laid-Open Application No. 2010-220855

### SUMMARY OF THE INVENTION

Since the estimation of body composition requires detailed information including the height, weight, gender, and age of a person to be measured as described above, even if an experience event for a measurement device is held, for example, in an event site or other places where many people gather, those who do not want to enter (report) detailed information would be reluctant to use the measurement device. Those who are reluctant to use a measurement device include those who are not health-conscious and those who are not confident in their bodies.

If a person does not enter the height, weight, or the like, body composition cannot be accurately estimated even if a measurement device is used to measure electric resistance values. As a result, such a person will be reluctant to use the measurement device.

A purpose of the disclosure made in view of the above problem is to provide a measurement device that can encourage a person to use the device even if the person is reluctant to measure body composition. Additionally, various kinds of data can be collected as a result.

A measurement device according to one aspect has: an electric resistance value acquisition unit for acquiring an electric resistance value of one or more parts of a body of a person to be measured; and a type determination unit for using the electric resistance value to determine a type representing a physical characteristic of the person to be measured from one or more predetermined points of view.

This configuration allows a person to be measured to find a result of determination of a type of the person's physical characteristic that cannot be found uniformly from information only on gender, age, or the like. As a result, different regression equations can be used or combined together according to the type. In addition, since a person to be measured can use the measurement device as though it were a game, those who are reluctant to measure body composition can be encouraged to use the measurement device.

In the above-described measurement device, the type determination unit may use the electric resistance value to determine the type independently of whether the type corresponds to an actual physical characteristic of the person to be measured or not.

The determination of a type that is independent of an actual physical characteristic of a person to be measured can encourage the person to be measured to use the measurement device as though it were a game.

In the above-described measurement device, the type determination unit may use the electric resistance value to determine the type set in a determination item that is for performing determination on a person to be measured from a predetermined point of view.

In the above-described measurement device, the determination item may be an index including at least one of gender, ethnicity, and evolution.

In the above-described measurement device, the determination item may be an index including at least one of a game character, an anime character, and a historical figure.

In the above-described measurement device, the type determination unit may indicate how much the body of the person to be measured belongs to which one of types, each defined similarly to the type, set in a determination item that is for performing determination on a person to be measured from a predetermined point of view.

Determining and indicating a type a person to be measured belongs to in these manners makes the person to be measured feel less reluctant to measure.

The above-described measurement device may have a body composition calculation unit for using the electric resistance value and an algorithm corresponding to the determined type to calculate a body composition value of the person to be measured.

This configuration enables body composition to be accurately calculated in accordance with a physical characteristic of a person to be measured.

The above-described measurement device may inform of the body composition value without informing of the determined type.

It may be more important for a person to be measured to find the body composition value than the type. In such a case, the measurement device may inform of the body composition value without informing of the type.

The above-described measurement device may have a divergence determination unit for comparing a body composition value corresponding to a body shape accepted as a choice from the person to be measured with the calculated body composition value, and determining a divergence from the body shape accepted as a choice.

This configuration allows a person to be measured to recognize the divergence between the person and an ideal body shape.

The above-described measurement device may inform of the determined divergence without displaying the calculated body composition value.

It may be more important for a person to be measured to find how large a divergence is from the person's ideal than the body composition value. In such a case, the measurement device may inform of the body composition value without displaying the body composition value.

The above-described measurement device can be implemented by causing a computer to read a program of one aspect and execute it. That is, the program is to cause a computer to function as a type determination unit that acquires, from a measurement device for measuring an electric resistance value of a body of a person to be measured, the electric resistance value and uses the electric resistance value to determine a type representing a physical characteristic of the person to be measured from one or more predetermined points of view.

With the use of these measurement devices, even a person who is reluctant to measure body composition can be encouraged to use the measurement devices. Additionally, data of people of various conditions can be collected as a result.

### BRIEF DESCRIPTION OF THE DRAWINGS

Figure 1 is a schematic configuration diagram of a measurement device of an embodiment of the invention;
Figure 2 is an example of a screen with a determination item for a generation characteristic;
Figure 3 is an example of a screen with a determination item for a gender characteristic;
Figure 4 is an example of a screen with a determination item for an evolution characteristic;
Figure 5 is a flowchart showing an example of processing in the measurement device of the invention;
Figure 6 is a schematic configuration diagram of a measurement device of Example 2;
Figure 7 is a flowchart showing an example of processing in the measurement device of Example 2;
Figure 8 is a schematic configuration diagram of a measurement device of Example 3;
Figure 9 is a flowchart showing an example of processing in the measurement device of Example 3;
Figure 10 is an example of a screen for a person to be measured to choose an ideal body shape; and
Figure 11 is an example of a screen showing a divergence in body composition value between a person to be measured and an ideal body shape.

### MODES OF EMBODYING THE INVENTION

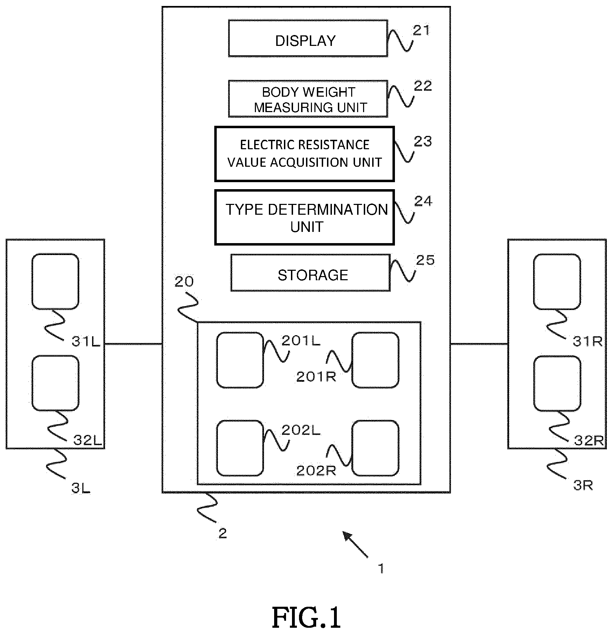

Figure 1 shows an example of a schematic configuration diagram of a measurement device of an embodiment of the disclosure. A measurement device 1 applies a small current to a person to be measured to measure an electric resistance value of the body such as impedance, reactance, and resistance.

The measurement device 1 comprises a main unit 2, a right handgrip 3R, and a left handgrip 3L. The main unit 2 comprises a platform 20 on which a person to be measured steps, a display 21, a body weight measuring unit 22, an electric resistance value acquisition unit 23, a type determination unit 24, and a storage 25. A person to be measured measures the body composition preferably by standing barefoot on the platform 20 and holding the right handgrip 3R and the left handgrip 3L with the right hand and the left hand, respectively, but only the platform 20 may be used without the use of the right handgrip 3R and the left handgrip 3L, or only the right handgrip 3R and the left handgrip 3L may be used without the use of the platform 20.

The platform 20 comprises a right-foot energizing electrode 201R, a right-foot measuring electrode 202R, a left-foot energizing electrode 201L, and a left-foot measuring electrode 202L. The right handgrip 3R comprises a right-hand energizing electrode 31R and a right-hand measuring electrode 32R, and the left handgrip 3L comprises a left-hand energizing electrode 31L and a left-hand measuring electrode 32L.

The display 21 is a display device for displaying a result of arithmetic processing performed by the type determination unit 24 or the like, and a device including, but not limited to, a liquid crystal display and an organic EL display, for example, can be used for it.

The body weight measuring unit 22 has a load cell for measuring the body weight of a person to be measured. The load cell comprises a flexure element made of a metallic member that becomes deformed according to a load, and a strain gauge affixed to the flexure element. When a person to be measured steps on the platform 20, the load of the person to be measured causes the flexure element of the load cell to bend and the strain gauge expands and contracts. The resistance value (the output value) of the strain gauge changes according to the expansion and contraction.

The electric resistance value acquisition unit 23 measures and acquires an electric resistance value based on a difference in potential that occurs in a current path measured by applying a small current of a predetermined frequency from each energizing electrode to a predetermined part of the body of a person to be measured, and on the current.

The measurement of an electric resistance value such as the impedance of the whole body and each body part of a person to be measured (the bioimpedance) is performed, for example, as follows.
(1) When the bioimpedance of the whole body is measured, a current is supplied by using the left-hand energizing electrode 31L and the left-foot energizing electrode 201L, and the difference in potential between the left-hand measuring electrode 32L being in contact with the left hand and the left-foot measuring electrode 202L being in contact with the left foot is measured in a current path where the current flows from the left hand through the left arm, the chest, the abdomen, and the left leg to the left foot.
(2) When the bioimpedance of the right leg is measured, a current is supplied by using the right-hand energizing electrode 31R and the right-foot energizing electrode 201R, and the difference in potential between the left-foot measuring electrode 202L being in contact with the left foot and the right-foot measuring electrode 202R being in contact with the right foot is measured in a current path where the current flows from the right hand through the right arm, the chest, the abdomen, and the right leg to the right foot.
(3) When the bioimpedance of the left leg is measured, a current is supplied by using the left-hand energizing electrode 31L and the left-foot energizing electrode 201L, and the difference in potential between the left-foot measuring electrode 202L being in contact with the left foot and the right-foot measuring electrode 202R being in contact with the right foot is measured in a current path where the current flows from the left hand through the left arm, the chest, the abdomen, and the left leg to the left foot.
(4) When the bioimpedance of the right arm is measured, a current is supplied by using the right-hand energizing electrode 31R and the right-foot energizing electrode 201R, and the difference in potential between the left-hand measuring electrode 32L being in contact with the left hand and the right-hand measuring electrode 32R being in contact with the right hand is measured in a current path where the current flows from the right hand through the right arm, the chest, the abdomen, and the right leg to the right foot.
(5) When the bioimpedance of the left arm is measured, a current is supplied by using the left-hand energizing electrode 31L and the left-foot energizing electrode 201L, and the difference in potential between the left-hand measuring electrode 32L being in contact with the left hand and the right-hand measuring electrode 32R being in contact with the right hand is measured in a current path where the current flows from the left hand through the left arm, the chest, the abdomen, and the left leg to the left foot.

The electric resistance value acquisition unit 23 applies a small current of one frequency, and may also apply currents of a plurality of frequencies such as low-frequency and highfrequency currents. In addition, not just impedance but reactance and resistance may be measured separately as electric resistance values.

The type determination unit 24 is implemented by a predetermined arithmetic processor in the main unit 2 executing a predetermined computer program. The storage 25 is also incorporated in the main unit 2, and holds the computer program to be executed by the arithmetic processor, data for arithmetic processing to be performed by the computer program, and the like. The storage 25 is made up of a storage medium such as an SSD and an HDD. The storage 25 may be a transportable storage medium that can be attached to and removed from the main unit 2. The arithmetic processor reads and writes information from and to the storage 25. The display 21, the arithmetic processor, and the storage device may integrate to be made up of a tablet computer, a smartphone, or the like. The computer program may be stored in the storage 25 in advance, may be downloaded via a network by the arithmetic processor and be stored in the storage 25, or may be stored in the storage 25 via a transportable storage medium.

The type determination unit 24 uses an electric resistance value of one or more parts of a person to be measured measured by the electric resistance value acquisition unit 23 to determine which type the person to be measured belongs to in a determination item that is for performing determination on a person to be measured from an aspect (type determination).

Determination items mentioned above are one or more items for performing determination on a person to be measured from an aspect such as gender, generation, ethnicity, and evolution. Types are groups into which people to be measured are classified in a determination item according to their electric resistance value. For example: types such as male and female are set in a determination item for gender in order to classify a person to be measured from an aspect of gender; types such as young age, middle age, and old age are set in a determination item for generation in order to classify a person to be measured from an aspect of generation; types such as Asian, Northern European, African American, and Arabian ethnicities are set in a determination item for ethnicity in order to classify a person to be measured from an aspect of ethnicity; and types such as ape-man, archanthropine, paleanthropine, neanthropine, and modern human are set in a determination item for evolution in order to classify a person to be measured from an aspect of evolutionary stage.

That is, since electric resistance characteristics of the body through which a small current is passed vary depending on physical characteristics including the length of a limb, the cross-sectional area of a muscle, a moisture ratio, and the density of muscle fibers, which type in a determination item a person to be measured belongs to can be determined by using a measured electric resistance value even without an input of information on the actual age, gender, height, weight, or the like of the person to be measured if groups different in physique characteristics or balance between muscle and fat can be classified as types in the determination item (groups classified by physical characteristics or more preferably by physique characteristics).

Therefore, on the basis of electric resistance values, such as impedance, reactance, and resistance, of one or more parts of a person to be measured measured with a plurality of frequencies, the type determination unit 24 compares values calculated by means of discrimination formulae of various physical characteristics prepared in advance with a threshold value for each type in a determination item stored in the storage described later, and determines which type in the determination item the person to be measured belongs to.

As for discrimination formulae of physical characteristics, discrimination formulae of various physical characteristics can be used, such as a discrimination formula of, for example, a characteristic based on the ratio of the length between a thin part and the body, a characteristic based on a muscle cross-sectional area balance of a limb, a characteristic based on the density of muscle fibers, the amount of water in a fat layer, a characteristic of the moisture distribution in a muscle, or the like.

As described above, determination items include, for example but not limited to, a determination item for generation, a determination item for gender, a determination item for evolution, and a determination item for ethnicity. The type determination unit 24 uses an electric resistance value measured from one or more parts of a person to be measured to determine a type representing a physical characteristic of the person to be measured from among a plurality of types set for each determination item. A type just has to be a group classified in a determination item according to an electric resistance value as described above, and therefore may be, for example, a group for categorizing a person to be measured from a viewpoint of body composition. A type which a person to be measured is determined to be does not always correspond to the person's actual physical characteristics including biological sex, and may not correspond to the person's actual physical characteristics. For example, there may be a case where a person to be measured is determined to be a female type in a determination item for gender even though the person is actually male, or where a person to be measured is determined to be an old age type in a determination item for age even though the person is actually in the person's twenties. Purposely determining an objectively obvious fact such as being male, being in one's twenties, and being Asian makes it enjoyable to see the gap between an objective fact and a type estimated from the body composition, and therefore even a person who is reluctant to use the measurement device 1 can be encouraged to use it as though it were a game. In addition, the determination of whether a person is male or female is seemingly meaningless since it is an obvious fact, but the determination of whether a person has a masculine body or a feminine body is meaningful for, for example, a person who is biologically male but is aiming to acquire feminine physical characteristics. Moreover, some Japanese people have dark skin and some Japanese people have fair skin, or even people of the same gender have different builds; for example, some women have a slight build and some women have a muscular build, and such people would be biologically lumped together as Japanese women according to their appearances. The determination of the type of a person to be measured based on the body composition (electric resistance value), however, can give the person a new awareness about the person's body. A determination item and types in it may be actually nonexistent, and therefore may be based on a physical characteristic of an actually nonexistent character such as an anime character and a game character. They may also be based on a physical characteristic of a person in the past such as a historical figure.

Types to which a person to be measured belongs may be clearly classified, for example, as male and female in the type determination performed by the type determination unit 24 as described above, but alternatively, for example, a ratio like 30% male and 70% female may be used in a determination item for gender, or the age of zero to a hundred may be used in a determination item for generation. In this way, the type determination unit 24 may determine how much a person corresponds to each type, such as the degree of correspondence to male, the degree of correspondence to female, the degree of correspondence to young age, the degree of correspondence to middle age, the degree of correspondence to old age, the degree of correspondence to Asian female, and the degree of correspondence to Euro-American male. In other words, the type determination unit 24 may determine the degree of correspondence to a type prepared in advance instead of determining which one of types prepared in advance a person belongs to.

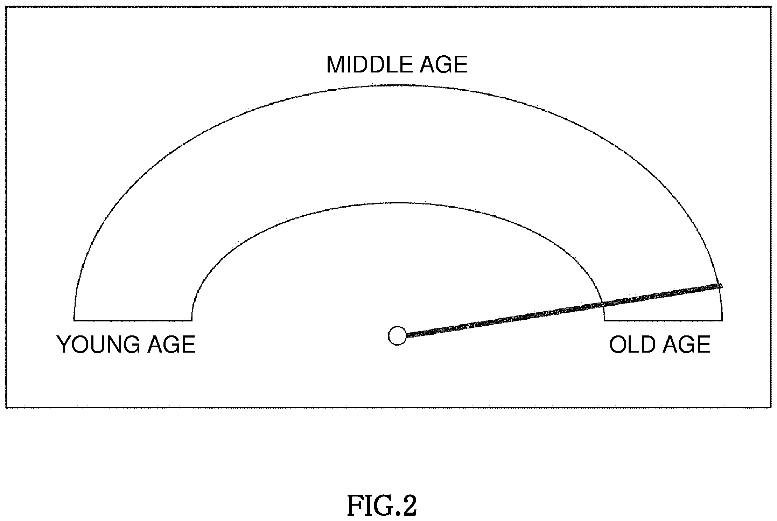

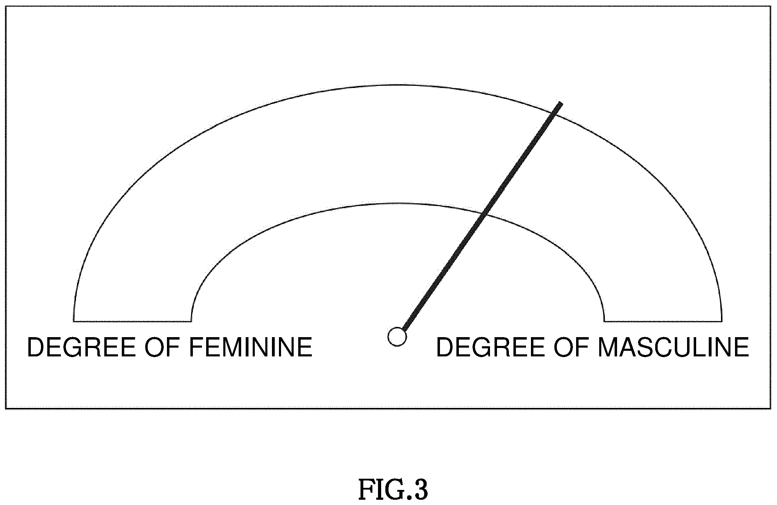

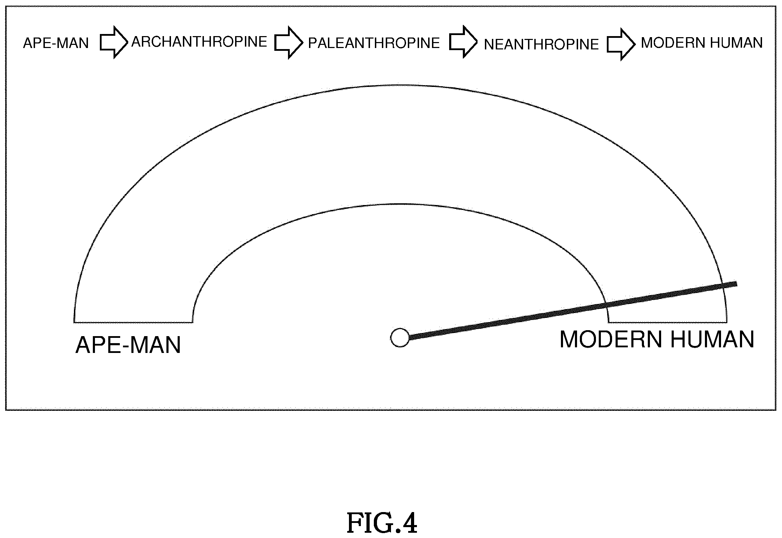

The type determination unit 24 may cause the display 21 to indicate which one of types in a determination item a person to be measured belongs to. Examples of this indication are shown in Figures 2 to 4. Figure 2 is an example of a screen with a determination item for generation such as young age, middle age, and old age. Figure 3 is an example of a screen with a determination item for gender such as male and female. Figure 4 is an example of a screen with a determination item for evolution such as ape-man, archanthropine, paleanthropine, neanthropine, and modern human. Note that electric resistance values can be calculated for ape-man, archanthropine, paleanthropine, neanthropine, and the like by modeling them from their skeletal specimens. While the types in each determination item are determined by a one-dimensional parameter in the examples of Figures 2 to 4, parameters for determining types may be two-dimensional or more.

If inputs of age, gender, height, and the like have been accepted from a person to be measured, the type determination unit 24 may use them to perform the above calculation. Body weight measured by the body weight measuring unit 22 may also be used to perform the above calculation.

The storage 25 holds various data required to perform processing in the measurement device 1 of the invention. For example, the storage 25 holds a computer program for performing arithmetic processing in the type determination unit 24, threshold values for types in each determination item for the type determination unit 24, and the like. For a determination item for generation, for example, the storage 25 holds a threshold value for a young age type, a threshold value for a middle age type, and a threshold value for an old age type. Moreover, for a determination item for gender, the storage 25 holds a threshold value for a male type and a threshold value for a female type. Furthermore, for a determination item for evolution, the storage 25 holds a threshold value for an ape-man type, a threshold value for a archanthropine type, a threshold value for a paleanthropine type, a threshold value for a neanthropine type, and a threshold value for a modern human type.

The measurement device 1 may have a function to communicate with another information processing device such as a server. While the measurement device 1 has the eight electrodes to be used for both hands and both feet, it may have only the four electrodes for both feet, only the four electrodes for both hands, or more electrodes than eight.

### Example 1

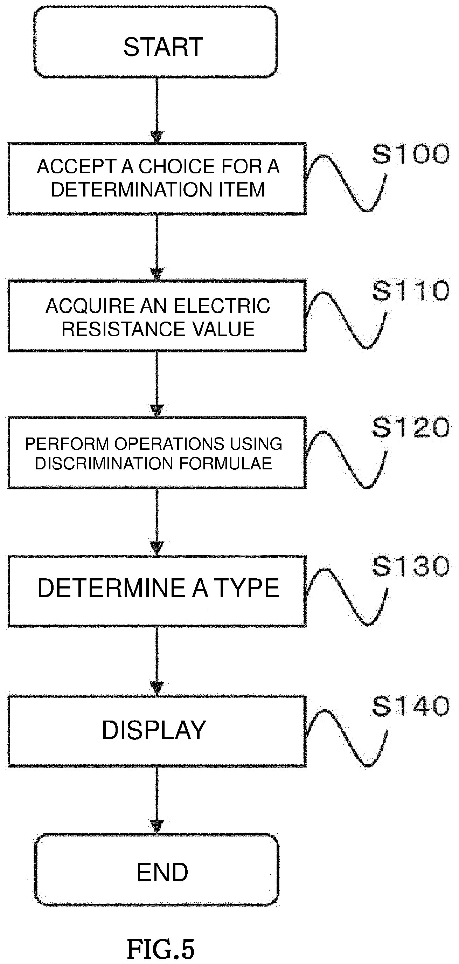

Now, an example of processing using the measurement device 1 of the invention will be described with reference to a flowchart of Figure 5.

The measurement device 1 causes the display 21 to show determination items, and accepts a choice about which determination item a determination is to be made for (S100).

A person to be measured steps barefoot on the platform 20 of the measurement device 1 in such a way that the right foot is placed on a right-leg energizing electrode and a right-leg measuring electrode, and that the left foot is placed on the left-foot energizing electrode 201L and the left-foot measuring electrode 202L. The right hand grips the right handgrip 3R, and the left hand grips the left handgrip 3L. The electric resistance value acquisition unit 23 applies a small current of one or more frequencies from each energizing electrode to a predetermined part of the body of the person to be measured, and measures the difference in potential occurring in the current pathway (S110). The electric resistance value acquisition unit 23 then calculates an electric resistance value based on the measured potential difference and the current. In addition, the body weight measuring unit 22 measures the body weight as required.

The type determination unit 24 substitutes the electric resistance value measured by the electric resistance value acquisition unit 23, plus the body weight according to conditions, into each discrimination formula stored in the storage 25, and thereby outputs the arithmetic result based on each discrimination formula (S120). Then, referring to the storage 25 and on the basis of threshold values for the types in the determination item a choice for which was accepted at S100 and of the arithmetic results obtained at S120, the type determination unit 24 determines which type in the determination item the person to be measured belongs to (S130).

For example, if the determination item a choice for which was accepted at S100 is generation and if the person to be measured is determined to be an old age type as a result of the determination made by the type determination unit 24 using discrimination formulae, the determination screen is displayed on the display 21 as shown in Figure 2 (S140).

A choice for a specific determination item need not be accepted at S100, and a type that the person to be measured belongs to may be determined for each of a plurality of predetermined determination items. In this case, which type the person to be measured belongs to is determined and indicated for each of the determination items for, for example, generation, gender, era, and ethnicity. Moreover, a choice for a specific determination item need not be accepted at S100, and a determination item may be accepted by causing the person to be measured to choose a determination item after an electric resistance value is measured by the electric resistance value acquisition unit 23.

The execution of such processing as described above can encourage even a person who is reluctant to use the measurement device 1 to voluntarily perform the measurement as though it were a game. As a result, even unwilling people's data that has been difficult to collect so far can be collected.

### Example 2

Another embodiment mode of the measurement device 1 of the invention will be described next. In this embodiment mode, a body composition value of a person to be measured is calculated by using a regression equation corresponding to a type in a determination item determined in, for example, Example 1.

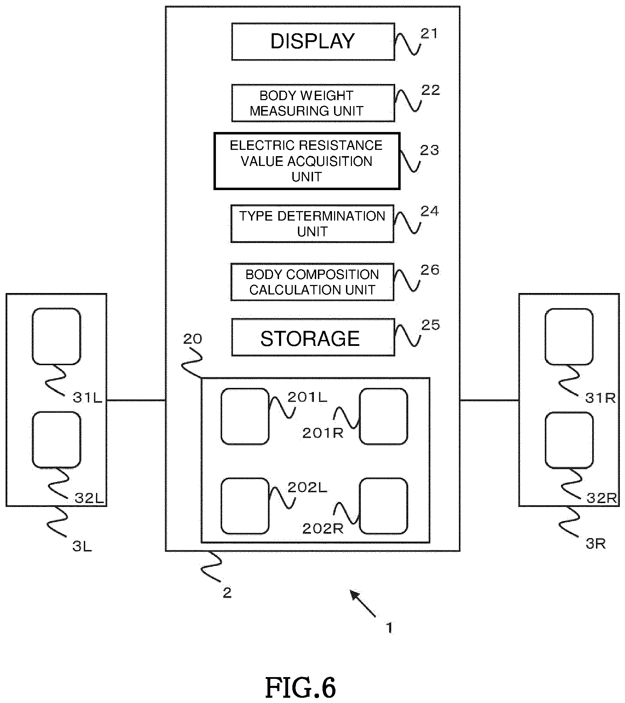

Figure 6 shows an example of a schematic configuration diagram of the measurement device 1 of the embodiment mode. The measurement device 1 of the embodiment mode further has a body composition calculation unit 26 in addition to the measurement device 1 of Figure 1.

The body composition calculation unit 26 calculates a body composition value of a person to be measured using a regression equation corresponding to the person's type in a determination item determined by the type determination unit 24. If the type determination unit 24 has determined one type, the body composition calculation unit 26 uses a regression equation for the type in the relevant determination item to calculate the body composition value. If the type determination unit 24 has determined types for a plurality of determination items, the body composition calculation unit 26 assigns weights to regression equations for the types in the determination items to specify how much they each are used, and calculates the body composition value of the person to be measured.

The storage 25 holds a regression equation for each type in each determination item. As for the regression equations, measurements of the bodies of various people, such as a measurement of an electric resistance value between predetermined parts of a body, are made to obtain a body composition, and regression equations calculated on the basis of the measurement values (electric resistance values) are stored corresponding to the types in each determination item. A regression equation may be stored for each type in each determination item, or regression equations may be stored corresponding to a plurality of determination items and a plurality of types. A regression equation may be stored for each combination of types in a plurality of determination items, and examples of such regression equations include a regression equation for Asian female (where a person belongs to an "Asian" type in a determination item for ethnicity and to a "female" type in a determination item for gender), a regression equation for Asian male (where a person belongs to an "Asian" type in a determination item for ethnicity and to a "male" type in a determination item for gender), a regression equation for Northern European female (where a person belongs to an "Northern European" type in a determination item for ethnicity and to a "female" type in a determination item for gender), a regression equation for Northern European male (where a person belongs to an "Northern European" type in a determination item for ethnicity and to a "male" type in a determination item for gender), a regression equation for African American female (where a person belongs to an "African American" type in a determination item for ethnicity and to a "female" type in a determination item for gender), a regression equation for African American male (where a person belongs to an "African American" type in a determination item for ethnicity and to a "male" type in a determination item for gender), a regression equation for Arabian female (where a person belongs to an "Arabian" type in a determination item for ethnicity and to a "female" type in a determination item for gender), and a regression equation for Arabian male (where a person belongs to an "Arabian" type in a determination item for ethnicity and to a "male" type in a determination item for gender).

The body composition calculation unit may use a predetermined algorithm corresponding to a type of a person to be measured determined by the type determination unit 24 to calculate a body composition value of the person to be measured. In addition to the above-described regression equations for calculating a body composition value using a measured electric resistance value, a machine learning model that accepts a measured electric resistance value as an input value and outputs a body composition value as an output value can also be used for this algorithm.

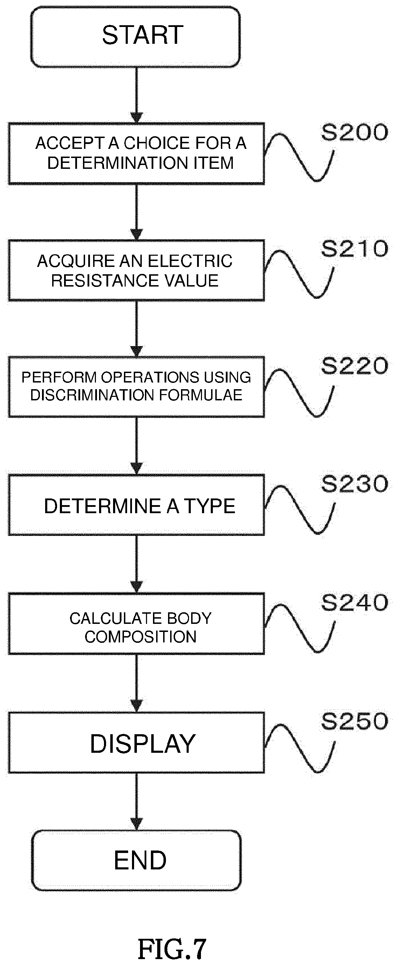

An example of processing using the measurement device 1 in the embodiment mode will be described next with reference to a flowchart of Figure 7. Steps S200 to S230 are the same as S100 to S130 of Example 1, and their descriptions are omitted.

The type determination unit 24 determines a type to which a person to be measured belongs for each determination item at S230, and then a regression equation for each type in the determination items stored in the storage 25 is specified. The specified regression equations are then used to calculate a body composition value of the person to be measured (S250).

For example, if a determination has been made only on a determination item for gender, the body composition calculation unit 26 uses a regression equation for the type to which the person to be measured belongs in the determination item for gender to calculate the body composition value of the person to be measured.

if determinations have been made on a plurality of determination items such as a determination item for gender and a determination item for ethnicity, and if the type determination unit 24 has determined the type of a person to be measured to be 80% Asian female, 15% Asian male, and 5% Arabian female, each ratio may be used as a weight assigned to each regression equation to calculate the body composition value. That is, adding together weighted values, namely, 80% of a value obtained from a regression equation for Asian female, 15% of a value obtained from a regression equation for Asian male, and 5% of a value obtained from a regression equation for Arabian female, or another predetermined operation is performed to calculate the body composition value of the person to be measured.

The body composition calculation unit 26 then causes the display 21 to show the body composition value of the determined person to be measured (S250).

The execution of the above-described processing enables a regression equation corresponding to a type in a determination item determined in Example 1 to be used to calculate and indicate a body composition value of a person to be measured. That is, even if an input including age, gender, height, weight, or the like is not accepted, the type determination unit 24 uses electric resistance values measured with a plurality of frequencies from one or more parts of a person to be measured to classify the person to be measured as a type in a determination item, and therefore the body composition calculation unit 26 can use a regression equation corresponding to the type in the determination item to perform arithmetic processing and calculate the body composition value of the person to be measured.

The present Example includes, but not limited to, using a regression equation corresponding to a type in a determination item determined in Example 1. For example, there is no need to indicate a definite type, and a body composition value of a person to be measured may be calculated by determining a physical characteristic and using a regression equation corresponding to it. Moreover, a type does not have to be determined in a determination item. Furthermore, a determined type need not be informed of, and the display 21 may be informed of and show a body composition value of a person to be measured.

### Example 3

Another embodiment mode of the measurement device 1 of the invention will be described next. In this embodiment mode, a body composition value of a person to be measured calculated in Example 2 is used to determine a divergence (distance) from the person's ideal body shape.

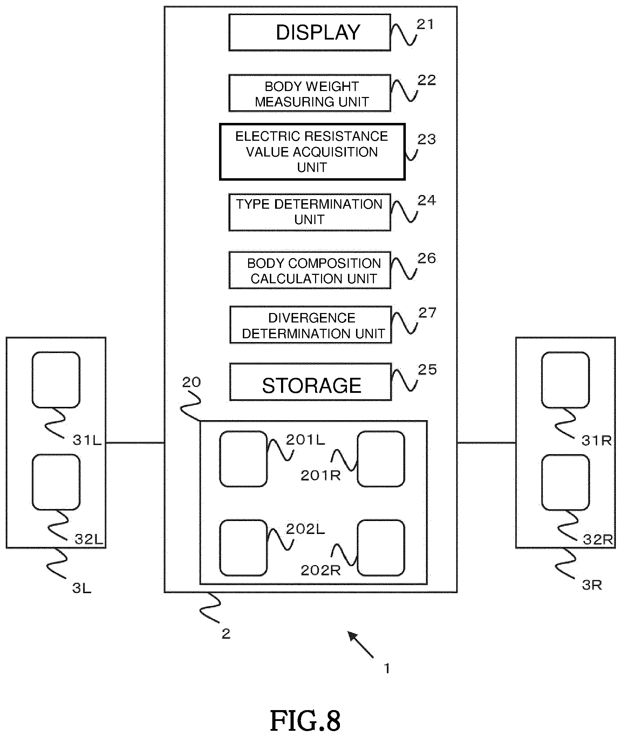

Figure 8 shows an example of a schematic configuration diagram of the measurement device 1 of the embodiment mode. The measurement device 1 of the embodiment mode further has a divergence determination unit 27 in addition to the measurement device 1 of Figure 6.

The divergence determination unit 27 uses a body composition value determined by the measurement device 1 to determine a divergence from a body composition value of a body shape that a person to be measured chose as an ideal.

The storage 25 further holds body composition values of a plurality of ideal body shapes as models. The storage 25 may also hold: the average range of body composition values for each of genders, generations, ethnicities, jobs, or other determination items and types or based on a predetermined criterion; a body composition value of a celebrity, historical figure, character, or the like; or the like.

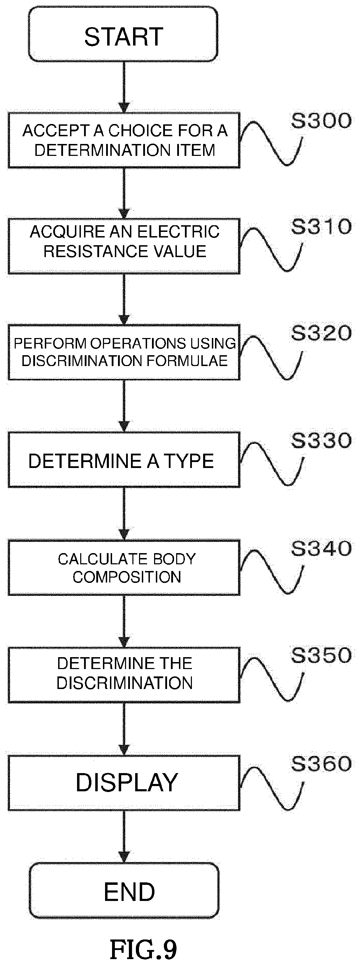

An example of processing using the measurement device 1 in the embodiment mode will be described next with reference to a flowchart of Figure 9. Steps S300 to S340 are the same as S200 to S240 of Example 2, and their descriptions are omitted.

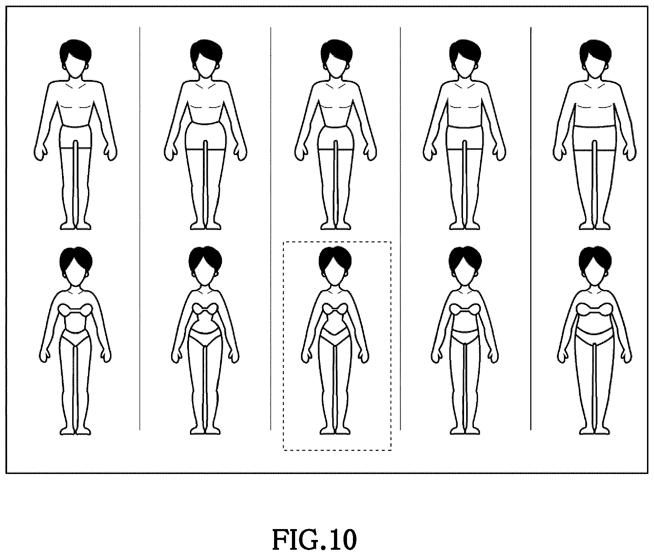

A person to be measured chooses the person's ideal body shape in one of the steps to S350, and the divergence determination unit 27 accepts it. For example, an ideal body shape is chosen from a body shape selection screen shown in Figure 10 and the choice is accepted. In Figure 10, an ideal body shape chosen by a person to be measured is indicated by an area with broken lines.

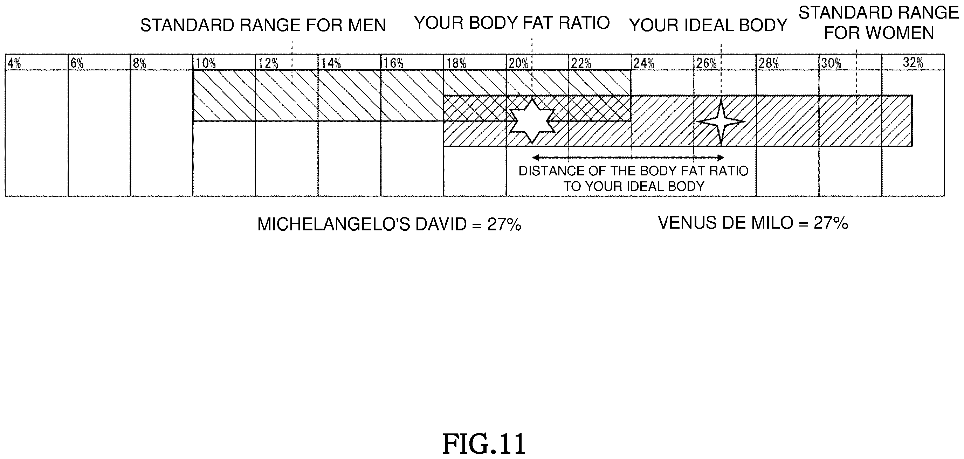

After the body composition calculation unit 26 calculates a body composition value of the person to be measured, a body composition value of the ideal body shape chosen by the person to be measured, such as the body fat level and the muscle mass level, is extracted from the storage 25, and the extracted value is compared with the body composition value calculated in S340, such as the body fat level and the muscle mass level, to calculate the divergence between them. The divergence is calculated by, for example, calculating the difference. The divergence determination unit 27 causes the display 21 to show the divergence calculated as above as shown in Figure 11 (S360). Figure 11 is a schematic diagram showing in a graphic form a divergence in body fat ratio, which is a body composition value, between a person to be measured and an ideal body shape chosen by the person to be measured. While Figure 11 shows a case in which the display includes the average range of body composition values for each gender, the display may also include: the average range of body composition values for each of generations, ethnicities, jobs, or other determination items and types; a body composition value of a celebrity or the like; or the like.

This allows the person to be measured to find the divergence in body composition value between the person and the ideal body shape.

While the body fat ratio of a person to be measured is displayed in Figure 11, it is all right to display the divergence only. In the case of Figure 11, for example, if the body fat ratio of a person to be measured is 21% and that of an ideal body shape chosen by the person to be measured is 27%, it is all right to display only the divergence (distance) from the ideal body shape using an expression such as "6% short to be your ideal body shape." This eliminates the display of the person's body fat ratio or other body composition values and makes it easier for the person to be measured to use the measurement device 1.

The calculated body composition value need not be shown, and information on the divergence determined by the divergence determination unit 27 may be informed of and be shown by the display 21.

### Example 4

As another example of the above-described embodiment modes, the functions of the type determination unit 24, the body composition calculation unit 26, the divergence determination unit 27, the storage 25, and the like are not provided in the measurement device 1, but may be implemented as an application program in a device different from the measurement device 1, such as a computer including a transportable communication terminal such as a smartphone and a tablet computer, by acquiring a measurement value measured by the measurement device 1. In this case, the measurement device 1 is provided with the electric resistance value acquisition unit 23, an electric resistance value measured by which is acquired by the above-described computer, and the same processing as described above is executed by the type determination unit 24, the body composition calculation unit 26, the divergence determination unit 27, and the like that are implemented by the application program of the computer.

As still another example of the above-described embodiment modes, processing just like fortune-telling may be provided by combining those with the birth date, the blood type, or the like of a person to be measured. In this case, a specific result may be output like fortune-telling by, for example, combining a type in a determination item, a body composition value, or the like of a person to be measured with information on the birth date, the blood type, or the like of the person to be measured to perform a predetermined operation.

As yet another example of the above-described embodiment modes, the athletic ability of a person to be measured may be acquired, and the acquisition result may be further combined with those to execute the processing of the type determination unit 24 or the like.

### INDUSTRIAL APPLICABILITY

With the use of the measurement device 1 of the invention, even a person who is reluctant to measure body composition can be encouraged to use the measurement device 1. Additionally, data of people of various conditions can be collected as a result.

### DESCRIPTION OF THE SYMBOLS

1: Measurement device
2: Main unit
3R: Right handgrip
3L: Left handgrip
20: Platform
21: Display
22: Body weight measuring unit
23: Electric resistance value acquisition unit
24: Type determination unit
25: Storage
26: Body composition calculation unit
27: Divergence determination unit
31R: Right-hand energizing electrode
31L: Left-hand energizing electrode
32R: Right-hand measuring electrode
32L: Left-hand energizing electrode
201 R: Right-foot energizing electrode
201 L: Left-foot energizing electrode
202R: Right-foot measuring electrode
202L: Left-foot measuring electrode

## Claims

1. A measurement device having:
an electric resistance value acquisition unit for acquiring an electric resistance value of one or more parts of a body of a person to be measured; and
a type determination unit for using the electric resistance value to determine a type representing a physical characteristic of the person to be measured from one or more predetermined points of view.

2. The measurement device according to claim 1,
wherein the type determination unit uses the electric resistance value to determine the type independently of whether the type corresponds to an actual physical characteristic of the person to be measured or not.

3. The measurement device according to claim 1 or 2,
wherein the type determination unit uses the electric resistance value to determine the type set in a determination item that is for performing determination on a person to be measured from a predetermined point of view.

4. The measurement device according to claim 3,
wherein the determination item is an index including at least one of gender, ethnicity, and evolution.

5. The measurement device according to claim 3,
wherein the determination item is an index including at least one of a game character, an anime character, and a historical figure.

6. The measurement device according to any of claims 1 to 5,
wherein the type determination unit indicates how much the body of the person to be measured belongs to which one of types, each defined similarly to the type, set in a determination item that is for performing determination on a person to be measured from a predetermined point of view.

7. The measurement device according to any of claims 1 to 6, having
a body composition calculation unit for using the electric resistance value and an algorithm corresponding to the determined type to calculate a body composition value of the person to be measured.

8. The measurement device according to claim 7, informing of the body composition value without informing of the determined type.

9. The measurement device according to any of claims 1 to 8, having
a divergence determination unit for comparing a body composition value corresponding to a body shape accepted as a choice from the person to be measured with the calculated body composition value, and determining a divergence from the body shape accepted as a choice.

10. The measurement device according to claim 9, informing of the determined divergence without displaying the calculated body composition value.

11. A program causing a computer to function as
a type determination unit that acquires, from a measurement device for measuring an electric resistance value of a body of a person to be measured, the electric resistance value and uses the electric resistance value to determine a type representing a physical characteristic of the person to be measured from one or more predetermined points of view.
